# EUROPEAN PATENT SPECIFICATION

(11) **EP 1 824 404 B1**
(45) Date of publication and mention of the grant of the patent: **15.08.2012**
(21) Application number: 05824872.5
(22) Date of filing: 18.11.2005
(51) Int. Cl.: A61B 18/14, A61B 17/11, A61B 17/115, A61M 25/10

(54) **DEVICES FOR ENERGY ASSISTED ARTERIO-VENOUS FISTULA CREATION**
VORRICHTUNGEN FÜR DIE ENERGIEGESTÜTZTE ERZEUGUNG EINER ARTERIOVENÖSEN FISTEL
DISPOSITIF DE FABRICATION D'UNE FISTULE ARTERIO-VEINEUSE ACTIONNEE PAR ENERGIE

(30) Priority: 22.11.2004 US 630385 P
(43) Date of publication of application: 29.08.2007
(73) Proprietor: Rox Medical Inc., San Clemente CA 92672 (US)
(72) Inventor: BRENNEMAN, Rodney, San Juan Capistrano, CA 92675 (US); SCHAEFER, Dean, A., Aliso Viejo, CA 92656 (US); FLAHERTY, J. Christopher, Topsfield, MA 01983 (US)
(74) Representative: Clark, Jane Anne
(86) International application number: PCT/US2005/041957
(87) International publication number: WO 2006/057920

(56) References cited:
- US-A1- 2004 059 280
- US-B1- 6 325 798
- US-B2- 6 503 247

## Description

### BACKGROUND OF THE INVENTION

The present invention relates generally to medical devices. More particularly, the present invention relates to devices for creating a flow of oxygenated blood into the venous system of a patient.

Chronic obstructive pulmonary disease affects millions of patients in the United States alone. The present standard of care is oxygen therapy, which requires a patient to remain near a stationary oxygen source or carry a bulky oxygen source when away from home or a treatment facility. It is easy to appreciate that such oxygen therapy has many disadvantages.

Lung reduction surgery has recently been proposed for treating patients with chronic pulmonary disease. Such surgery, however, is not a panacea. It can be used on only a small percentage of the total patient population, requires long recovery times, and does not always provide a clear patient benefit. Even when successful, patients often continue to require supplemental oxygen therapy.

There is therefore a need for improved approaches, including both devices and methods, for treating patients suffering from chronic obstructive pulmonary disease. If would be desirable if such devices and methods were also useful for treating patients with other conditions, such as congestive heart failure, hypertension, lung fibrosis, adult respiratory distress syndrome, and the like. Such devices and methods should provide for effective therapy, preferably eliminating the need for supplemental oxygen therapy in the treatment of chronic obstructive pulmonary disease. Utilization of the systems, devices and methods of the present invention will require long-term efficacy, including chronic patency of any fistula created to provide long-term therapeutic benefit. At lease some of these objectives will be met by the invention described hereinafter.

Figure 4a of US 2004/0059280 describes an approach in which a catheter is advanced transluminally into an artery and a tissue-penetrating element is passed from the catheter to form a first passageway through the wall of the artery. After a first blood flow passageway has been created in this manner, a guidewire may be passed through the tissue-penetrating element or through the catheter and through the newly-created first passageway. Thereafter the tissue-penetrating element is deactivated (e.g. retracted into the catheter) and the catheter is advanced over the guidewire, through the first passageway, and into the lumen of the vein past the site of the obstruction in the adjacent artery. Thereafter, with the distal portion of the catheter positioned within the lumen of the vein, the tissue-penetrating element is once again advanced out of the catheter to form a second blood flow passageway which extends through the wall of the vein, any tissue located between the vein and the artery, and through the wall of the artery. Thereafter, the tissue-penetrating element may be once again retracted into the catheter and the catheter may be retracted from the vasculature and out of the body. In this manner, formation of a first blood flow passageway upstream of the arterial obstruction and a second blood flow passageway downstream of the arterial obstruction is accomplished.

### BRIEF SUMMARY OF THE INVENTION

The present invention is set out in the appended claims. The present disclosure includes a catheter apparatus is adapted for insertion over a guidewire that has been placed between two anatomical structures, typically an artery and a vein. The catheter apparatus comprises a first elongate tube and a second elongate tube. The first elongate tube has a proximal end, a distal end and a lumen therebetween. The second elongate tube has a proximal end, a distal end which is usually tapered, and a lumen therebetween. By "tapered," it is meant that the distal end of the second elongate tube will have a reduced width or diameter at the distal-most tip when compared to the width or diameter of the main body of the second elongate tube. While the taper will preferably be regular, e.g., having a conical geometry, it could also have other symmetrical and non-symmetrical shapes. For example, the surface could have a generally "concave" configuration where the ramp from the distal tip to the main body of the second elongate tube has an increasingly steep slope in the proximal direction. Alternatively, it could have a generally "convex" geometry where the slope of the increase in width or diameter decreases in the proximal direction. Various asymmetric configurations where the tip might be radially offset at the distal-most end are also possible. For example, the port which receives the guidewire at the distal-most tip of the second elongate tube may be radially offset so that it is aligned with the periphery of the main body of the second elongate tube rather than the axis. The second elongate tube is configured to be slidingly received by the lumen of the first elongate tube. An ablation element for creating and/or modifying a fistula between the two anatomical structures after the two anatomical structures are brought together is provided, typically on at least one of the distal end of the first elongate tube and the distal end of the second elongate tube. In the exemplary embodiments, the ablation element is found on the tapered end of the second elongate tube so that it may enter the fistula in order to help create the fistula and/or modify the tissue surrounding the fistula in a beneficial manner.

The anatomical structures are preferably an artery and a vein, the artery preferably being selected from the group consisting of: the aorta; the femoral arteries; the iliac arteries; the brachial arteries; the carotid arteries; the subclavian arteries; the mammary arteries; the renal arteries; and the hepatic artery. The vein is preferably selected from the group consisting of: the superior vena cava (SVC); inferior vena cava (IVC); the femoral veins; the iliac veins; the brachial veins; the radial veins; the jugular veins; and the mammary veins. The vessels are preferably a minimum of 4 mm in diameter. The fistula may be created to provide a source of oxygenated blood into the venous system, such that the oxygen content of blood entering the lungs is elevated due to the fistula, this elevation providing a therapeutic benefit to patients with multiple forms of heart disease.

In an exemplary embodiment, the apparatus includes an anastomotic clip which is selected to perform at least one of the following functions: providing tension between walls of the two anatomical structures; providing hemostasis in or around the fistula tissue; reducing neointimal proliferation into the fistula; exerting radial force on the tissue surrounding the fistula; acting as a radioactive source to deliver radiation to the tissue surrounding the fistula; acting as a drug delivery depot delivering one or more agents to the tissue of the fistula; and combinations thereof. In an alternate or additional embodiments, the clip may function as an ablation element, such as an electrode to deliver monopolar or bipolar RF energy.

Ablation elements can be placed on tips, such as dilating conical tips at the distal end of the elongate tubes, on the surface of an integral balloon, or on an outer wall of an elongate tube. Ablation elements can be configured to deliver energy in the form of: sound energy such as acoustic energy and ultrasound energy; electromagnetic energy such as electrical, magnetic, microwave and radiofrequency energies; thermal energy such as heat and cryogenic energies; chemical energy; light energy such as infrared and visible light energies; mechanical energy; radiation; and combinations thereof. In preferred embodiments, multiple forms of energy can be delivered by the catheter apparatus, such as multiple forms of energy delivered by a single ablation element.

Described herein is a system for creating a fistula between two anatomical structures. The system includes one or more catheter apparatus as set out above and an energy source for providing energy to one or more ablation elements of the catheter apparatus. The energy provided is selected from the group consisting of: sound energy such as acoustic energy and ultrasound energy; electromagnetic energy such as electrical, magnetic, microwave and radiofrequency energies; thermal energy such as heat and cryogenic energies; chemical energy; light energy such as infrared and visible light energies; mechanical energy; radiation; and combinations thereof. The system may further comprise an imaging device, such as an intravascular ultrasound (IVUS) catheter insertable into a lumen of the catheter apparatus, for providing a cross-sectional image to the operator of the fistula, any implants, and the tissue surrounding the fistula. The system may further include additional devices to support the creation of and/or modify the fistula, as well as perform other functions. These additional devices are selected from the group consisting of: a balloon catheter such as a balloon catheter used to increase the diameter of the clip and/or the fistula; a drug delivery catheter such as a balloon eluding drug delivery catheter used to deliver one or more agents to the clip and/or the fistula; a radiation delivery catheter such as a radiation delivery catheter used to reduce neointimal proliferation into the fistula; an energy delivery catheter such as an energy delivery catheter used to apply energy to tissue protruding into the fistula; a cutting device such as a pull-back cutter used to remove tissue that is protruding into the fistula; a manipulating arm such as a manipulating ann used to reposition and/or reconfigure the anastomotic clip; a clip delivery device such as a catheter used to place a second anastomotic clip; and combinations thereof.

Described herein is a catheter apparatus for insertion over a guidewire which has been placed between two anatomical structures comprises a first elongate tube, a second elongate tube, and a third elongate tube. The first elongate tube includes a proximal end, a distal end, and a lumen therebetween. The second elongate tube includes a proximal end, a conically tapered distal end, and a lumen therebetween. The second elongate tube is slidingly received by the lumen of the first elongate tube. The third elongate tube includes a proximal end and a distal end, as well as an ablation element for creating and/or modifying a fistula between the two anatomical structures. In a specific embodiment, the third elongate tube includes a spiral shaped ablation element on its distal end. In another specific embodiment, the second elongate tube includes an ablation element. In another specific embodiment, a pull wire is attached to a distal portion of the first elongate tube or the second elongate tube. In another preferred embodiment, the catheter apparatus includes a deployable anastomotic clip.

Described herein is a catheter apparatus for insertion over a guidewire which has been placed between two anatomical structures. The catheter apparatus comprises a first elongate tube and a second elongate tube. The first elongate tube includes a proximal end, a distal end, and a lumen therebetween. The second elongate tube includes a proximal end, a conically tapered distal end, and a lumen therebetween. The second elongate tube is slidingly received by the lumen of the first elongate tube. The first elongate tube includes on its distal end an ablation element for creating and/or modifying a fistula between two anatomical structures. In a preferred embodiment, the conical tip of the second elongate tube also includes an ablation element. In another preferred embodiment, the catheter apparatus includes a deployable anastomotic clip.

Described herein is a catheter apparatus for creating a fistula between a first anatomical structure and a second anatomical structure. The catheter apparatus comprises a first elongate tube, a second elongate tube and a third elongate tube. The first elongate tube includes a proximal end, a distal end, and a lumen therebetween. The second elongate tube includes a proximal end, a conically tapered distal end, and a lumen therebetween. The second elongate tube is slidingly received by the lumen of the first elongate tube. The third elongate tube includes a proximal end, a sharpened distal tip, and a lumen therebetween. The third elongate tube is configured to puncture through a wall of the first anatomical structure and a wall of the second anatomical structure such that a guidewire can be advanced through the lumen of the third elongate tube from the first anatomical structure to the second anatomical structure. In a preferred embodiment, the third elongate tube is slidingly received by the lumen of the first elongate tube. In an alternative preferred embodiment, the third elongate tube is slidingly received by the lumen of the second elongate tube. In another preferred embodiment, the conical end of the second elongate tube includes an ablation element.

Described herein is a catheter apparatus for insertion over a guidewire which has been placed between two anatomical structures . The catheter apparatus comprises a first elongate tube and a second elongate tube. The first elongate tube includes a proximal end, a distal end, a lumen therebetween, and an expandable balloon near its distal end. The second elongate tube includes a proximal end, a distal end, a lumen therebetween, and an expandable balloon near its distal end. The second elongate tube is slidingly received by the lumen of the first elongate tube. Each balloon includes a proximal surface and a distal surface. The balloon of the first elongate tube includes an ablation element on its distal surface. The balloon of the second elongate tube includes an ablation element its proximal surface. In a preferred embodiment, at least one ablation element is a conductive coating on the balloon. In another preferred embodiment, at least one ablation element is a flexible conductive plate affixed to the balloon.

Described herein is a catheter apparatus for insertion over a guidewire which has been placed between two anatomical structures. The catheter apparatus comprises an elongate tube with a proximal end, a distal end, a lumen therebetween, and an inflatable balloon with an outer surface. At least one ablation element is mounted to the outer surface of the balloon. The ablation element is configured to transmit energy for creating and/or modifying a fistula between two anatomical structures.

Disclosed herein is a therapeutic method for treating a patient. The method comprises diverting a flow of oxygenated arterial blood to the venous system by having for a long-term period a fistula between a first anatomical structure and a second anatomical structure, such as an artery and a vein. The fistula is created with an apparatus configured to deliver energy to the tissue surrounding the fistula while creating and/or modifying the fistula. Contrast medium is optionally delivered from the apparatus to confirm that the apparatus has reached the second anatomical structure. In a preferred embodiment, energy is delivered to assist in passing a device from a starting vessel to a target vessel. In another preferred embodiment, the method further includes the step of placing an anastomotic clip in the fistula. In another preferred embodiment, the clip provides a function selected from the group consisting of: preventing bleeding in or around the fistula; providing tension between an artery and a vein; scaffolding the fistula in a radially outward direction; preventing tissue from residing within the flow path of the fistula; preventing proliferation of tissue into the flow path of the fistula; and combinations thereof. In another preferred embodiment, the therapy is a respiratory or cardio-respiratory therapy. In another preferred embodiment, the therapy is a cardiac therapy. In another preferred embodiment, the therapy is a circulatory therapy. In another preferred embodiment, an additional device is placed into the fistula over a guidewire inserted through the fistula. The additional device can be selected from the group consisting of: a balloon catheter such as a balloon catheter used to increase the diameter of the clip and/or the fistula; a drug delivery catheter such as a balloon eluding drug delivery catheter used to deliver one or more agents to the clip and/or the fistula; a radiation delivery catheter such as a radiation delivery catheter used to reduce neointimal proliferation into the fistula; an energy delivery catheter such as an energy delivery catheter used to apply energy to tissue protruding into the fistula; a cutting device such as a pull-back cutter used to remove tissue that is protruding into the fistula; a manipulating arm such as a manipulating arm used to reposition and/or reconfigure the anastomotic clip; a clip delivery device such as a catheter used to place a second anastomotic clip; and combinations thereof. The methods do not constitute a part of the invention.

Described herein is also an examplary therapeutic method for treating a patient. The method comprises diverting a flow of oxygenated arterial blood to the venous system by having for a long-term period a fistula between a first anatomical structure and a second anatomical structure, such as an artery and a vein. The fistula is modified with an apparatus configured to deliver energy to the tissue surrounding the fistula after the fistula has been created. In a preferred embodiment, energy is delivered to treat the fistula. In another preferred embodiment, the method further includes the step of placing an anastomotic clip in the fistula. In another preferred embodiment, the clip provides a function selected from the group consisting of: preventing bleeding in or around the fistula; providing tension between an artery and a vein; scaffolding the fistula in a radially outward direction; preventing tissue from residing within the flow path of the fistula; preventing proliferation of tissue into the flow path of the fistula; and combinations thereof. In another preferred embodiment, the therapy is a respiratory or cardio-respiratory therapy. In another preferred embodiment, the therapy is a cardiac therapy. In another preferred embodiment, the therapy is a circulatory therapy. In another preferred embodiment, an additional device is placed into the fistula over a guidewire inserted through the fistula. The additional device can be selected from the group consisting of: a balloon catheter such as a balloon catheter used to increase the diameter of the clip and/or the fistula; a drug delivery catheter such as a balloon eluding drug delivery catheter used to deliver one or more agents to the clip and/or the fistula; a radiation delivery catheter such as a radiation delivery catheter used to reduce neointimal proliferation into the fistula; an energy delivery catheter such as an energy delivery catheter used to apply energy to tissue protruding into the fistula; a cutting device such as a pull-back cutter used to remove tissue that is protruding into the fistula; a manipulating arm such as a manipulating arm used to reposition and/or reconfigure the anastomotic clip; a clip delivery device such as a catheter used to place a second anastomotic clip; and combinations thereof.

Described herein is a kit for creating a fistula between a first anatomical structure and a second anatomical structure. The kit includes a catheter apparatus comprising a first elongate tube, a second elongate tube, and a third elongate tube. The first elongate tube includes a proximal end, a distal end and a lumen therebetween. The second elongate tube includes a proximal end, a conically tapered distal end, and a lumen therebetween. The second elongate tube is sized and configured to be slidingly advanced and retracted within the lumen of the first elongate tube. The third elongate tube includes a proximal end, a conically tapered distal end, and a lumen therebetween. The third elongate tube is also sized and configured to be slidingly advanced and retracted within the lumen of the first elongate tube. The conically tapered distal end of either the second or third elongate tube includes an ablation element for creating and/or modifying a fistula between two anatomical structures.

Both the foregoing general description and the following detailed description are exemplary and are intended to provide further explanation of the embodiments of the invention as claimed.

### BRIEF DESCRIPTION OF THE DRAWINGS

The accompanying drawings, which are incorporated in and constitute a part of this specification, illustrate various embodiments of the present, disclosure and, together with the description, serve to explain the principles of the invention. In the drawings:

Fig. 1 illustrates a system including a catheter apparatus;

Fig. 1a illustrates a sectional view of the distal end of the catheter apparatus of Fig. 1;

Figs. 1b through 1d illustrate alternative tapered tip geometries to the second elongate tube;

Fig. 2 illustrates the catheter apparatus placed into the vasculature of a patient to create a fistula between the aorta and the IVC at a location immediately superior to the aortic bifurcation;

Fig. 3 illustrates a sectional view of the distale end of an example of the catheter apparatus;

Fig. 4 illustrates a sectional view of the distal end of a preferred embodiment of the catheter apparatus;

Figs. 5a through 5e illustrates a sectional view of a preferred embodiment of a fistula creation apparatus and method including the steps of passing a guidewire from a starting vessel to a target vessel, creating a fistula, and placing an anastomotic clip in the fistula;

Fig. 6 illustrates a sectional view of a catheter apparatus including an outer shaft with an expandable balloon and an inner shaft with an expandable balloon, each balloon including an ablation element on its surface

Fig. 6a illustrates a sectional view of the catheter apparatus of Fig. 6 placed from a starting vessel to a target vessel prior to creating or modifying a fistula;

Fig. 7 illustrates a catheter apparatus with an expandable balloon, the balloon including an ablation element.

### DETAILED DESCRIPTION OF THE INVENTION

Reference will now be made in detail to examples illustrated in the accompanying drawings. Wherever possible, the same reference numbers will be used throughout the drawings to refer to the same or like parts.

Blood flows from the heart via the arterial system to the vasculature of the tissue from which it returns back to the heart via the venous system. Blood returning to the right side of the heart is pumped to the lungs where it becomes oxygenated or re-oxygenated, before returning to the left side of the heart to be pumped to the body's tissues via the arterial system. Blood flow experiences a resistance from all of the systemic vasculature, which is referred to as the systemic vascular resistance (SVR). SVR excludes the pulmonary vasculature, but when these two are combined it is sometimes referred to as total peripheral resistance (TPR). SVR is determined by factors that influence vascular resistance in individual vascular beds. Mechanisms that cause vasoconstriction (reducing the caliber of a vessel) will increase SVR, and those that cause vasodilation (increasing the caliber of a vessel) will decrease SVR. The actual change in SVR in response to neurohumoral activation, for example, will depend up on the degree of activation and vasoconstriction, the number of vascular beds involved, and the relative in-series and parallel arrangements of these vascular beds to each other. Although SVR is primarily determined by changes in blood vessel diameters, changes in blood viscosity will also affect SVR.

As described herein, SVR is decreased by having an arteriovenous fistula that re-circulates blood from the arterial system to the venous system. The re-circulated blood through the fistula bypasses the peripheral microcirculation and decreases the SVR. In general, the fistula may be created between a large (proximal) artery and a large (proximal) vein, such as vessels with diameters greater than 4 mm. The location is selected to shunt and (quickly) re-circulate blood from the high resistance arterial system with a high oxygen concentration to the low resistance venous system with a low oxygen concentration.

The fistula is created between the aorta and the IVC, either proximal of the renal arterial, or distal of the renal arteries. In alternative embodiments, the artery in which the fistula is created is selected from the group consisting of: the femoral arteries; the iliac arteries; the axillary arteries; the brachial arteries; the radial arteries; the carotid arteries; the subclavian arteries; the mammary arteries; the renal arteries; the hepatic artery; and a combination of one or more of these arteries. The vein in which the fistula is created is selected from the group consisting of: the SVC; IVC; the femoral veins; the iliac veins; the axillary veins; the brachial veins; the radial veins; the jugular veins; the mammary veins; and a combination of one or more of these veins. In a preferred embodiment, the artery and vein chosen, as well as the fistula diameter, are chosen to create a blood flow rate of at least 5 ml/min. Fistula length preferably ranges from about 2.5 mm to about 15 mm, and the radius preferably ranges from about 2.5 mm to about 15 mm. One could also express the lumen opening (in the wall of each vessel) in the terms of cross sectional area, which would range from about 19 mm² to about 750 mm².

With respect to the respiratory effects, an important consequence of shunting arterial blood to the venous circulation (such as the aorta to the IVC) is that blood with high O₂ content circulates to the venous blood system without having the O₂ extracted in tissue capillaries. The O₂ "rich" arterial blood re-circulates to, and mixes with, the low O₂ concentration of the venous system. As a result, the blood flowing through the fistula of the present invention increases the O₂ concentration in the venous blood. The increase of O₂ concentration in the venous blood system leads to an increase in the O₂ concentration in the arterial blood in two ways. First, since the blood that is shunted does not have O₂ extracted by tissue capillaries, the blood returning to the lungs has a higher O₂ concentration after the creation of the shunt than before. Second, O₂ is carried in the blood in two forms: (i) dissolved in arterial plasma, and (ii) bound to a protein called hemoglobin that is contained in red blood cells. Oxygen binds to hemoglobin with curvilinear kinetics, so that O₂ very efficiently binds to (and is carried by) hemoglobin at high Pa O₂ (partial pressure of O₂ in arterial plasma), but when the Pa O₂ is low (in particular below a Pa O₂ of 60 mmHg), O₂ is less efficiently bound to (or carried by) hemoglobin. Since the amount of O₂ that is bound to hemoglobin is related to the Pa O₂, an increase in Pa O₂ will result in greater binding of O₂ to hemoglobin, and increased oxygen carrying capacity.

With respect to circulatory effects, an important consequence of decreasing SVR is related to the fact that the lungs regulate their blood flow according to the O₂ content. A low O₂ content in the small pulmonary arteries impairs blood flow to the lung resulting in a high pulmonary pressure - a process call hypoxic pulmonary vasoconstriction. Therefore increasing the O₂ content in the small pulmonary arterial blood should decrease the pulmonary arterial blood pressure. Other important circulatory consequences, as described hereabove with respect to cardiac consequences, are a decrease in systemic arterial blood pressure, a decrease in systemic arterial systolic pressure and/or a decrease in systemic arterial diastolic pressure.

The different distinct effects could be beneficial to patients with cardiac problems as a cardiac therapy, to patients with respiratory problems as a respiratory or cardio-respiratory therapy, or to patients with circulatory problems as a circulatory therapy. An illustrative list of therapies is for instance:

*Cardiac therapies:* The fistula and fistula creation apparatus could benefit patients with cardiac failure or patients who suffer from a low cardiac output (congestive heart failure) by providing an increased cardiac output.

*Respiratory or cardio-respiratory therapies:* The fistula and fistula creation apparatus could benefit patients with pulmonary arterial hypertension by lowering pulmonary arterial blood pressure, patients with heart and/or respiratory failure by increasing arterial oxygen concentration, patients with chronic obstructive pulmonary disease by increasing of blood oxygen concentration.

*Circulatory therapies:* The fistula and fistula creation apparatus could benefit patients with hypertension by lowering systemic arterial, systolic and/or diastolic blood pressure.

Other diseases or conditions that could benefit are, for instance, hypotension (by increasing cardiac output), lung fibrosis, adult respiratory distress syndrome, and the like.

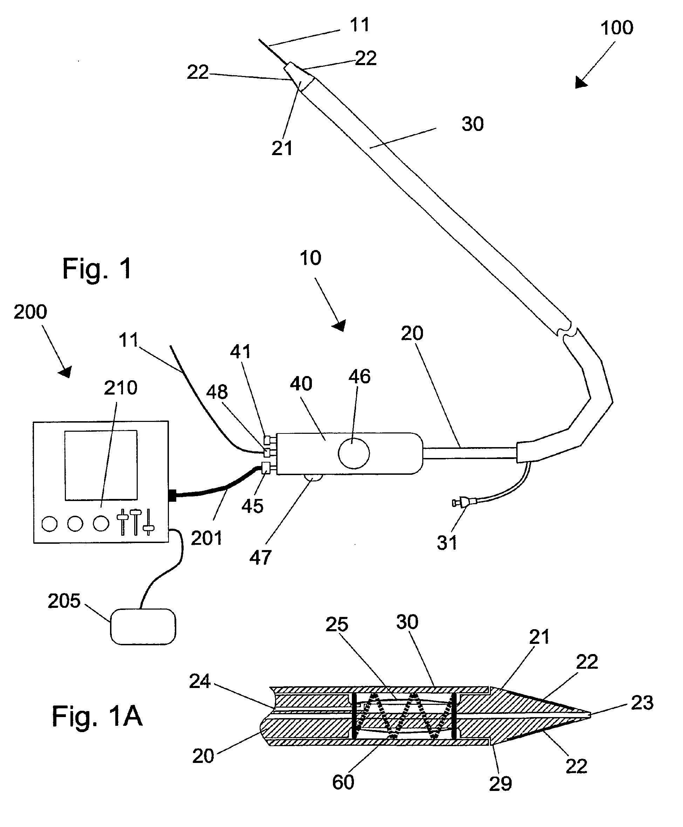

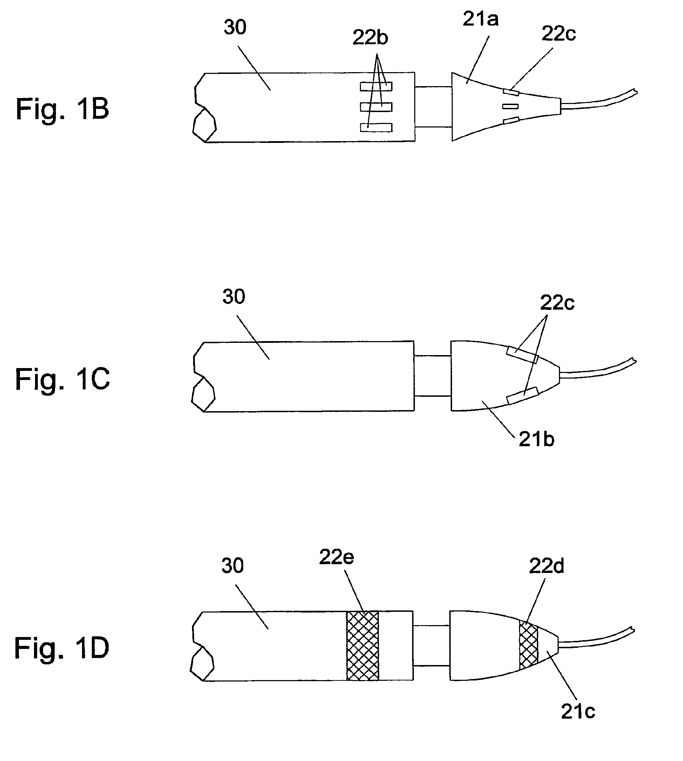

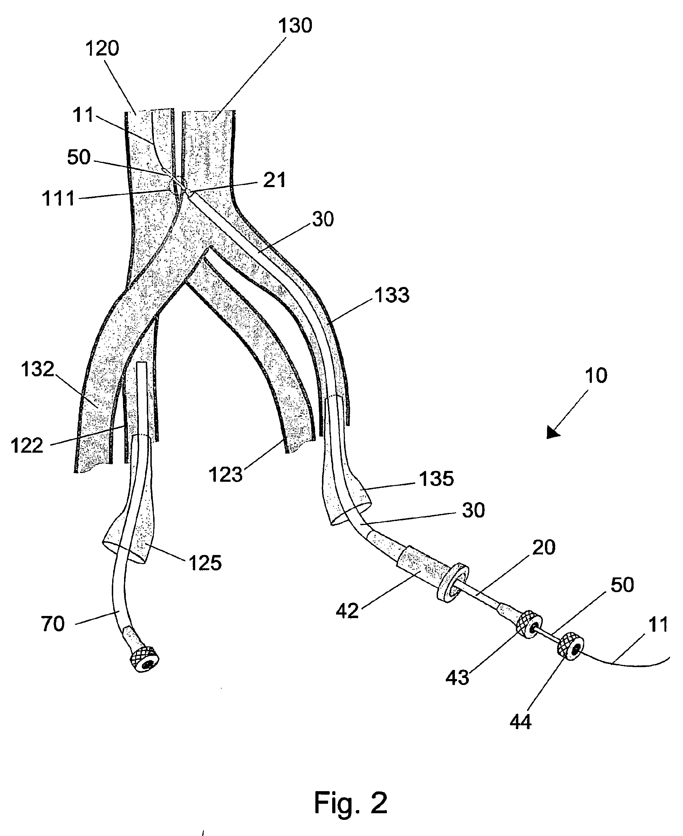

Referring now to Fig. 1, a system and catheter apparatus is illustrated. System 100 includes catheter apparatus 10, which is configured to be placed intravascularly over a guidewire 11 that has previously been placed between an artery and a vein, such as the aorta and the IVC (Fig. 2). Catheter apparatus 10 includes a first elongate tube, outer sheath 30, which includes a proximal end, a distal end, and a lumen therethrough. Outer sheath 30, and other tubular conduits of catheter apparatus 10 are constructed of biocompatible materials such as polyether block amides (Pebax™); polyimides; polyurethanes; silicones; nylons; polyvinyl chloride (PVC); polyesters; and combinations thereof. The materials used and other construction parameters are chosen to provide suitable flexibility and column strength to be percutaneously and transluminally introduced into the patient as well as perform other functions such as crossing from a starting vessel to a target vessel. These types of elongate tubes may be constructed of an outer layer, an inner layer and a braid residing therebetween. The braid may be constructed of various materials including stainless steel; nickel-titanium alloy (e.g., Nitinol™); monofilament fiber; polymers; and combinations thereof. In addition, these types of elongate tubes may include a pull wire, such as a pull wire operably attached to control means on the proximal end of the apparatus. Outer sheath 30 includes, near its proximal end, flush port 31 which is configured to be attachable to a flushing syringe, used to flush the space between outer sheath 30 and inner core 20.

Inner core 20 is another (second) elongate tube, having a proximal end, a conically or otherwise tapered distal end, and a lumen therebetween. Inner core 20 is configured to be slidingly advanced and retracted within the lumen of outer sheath 30. Located on the distal end of inner core 20 is a tissue dilating tip, conical tip 21 (Fig. 1a) which also includes a lumen 23 and is preferably of a construction that is more rigid than that of inner core 20. Although illustrated as a separate component, the conical tip 21 may also be formed integrally with the remaining portion of the inner core 20. Even when integrally formed, the polymer used to form the conical tip 21 may be formulated to have an increased hardness relative to the remaining portion of the inner core 20. Catheter apparatus 10 includes, at its proximal end, handle 40, which is attached to inner core 20. Handle 40 includes a guidewire insertion port, port 48. In Fig. 1, catheter apparatus 10 is depicted with guidewire 11 entering a lumen of inner core 10 through port 28 of handle 40, and exiting inner core 20 at the distal end of conical tip 21. Included on the outer surface of conical tip 21 is one or more tissue ablating elements, electrodes 22, which are configured to deliver one or more forms of energy in or around the fistula of the present invention. The energy can be delivered for a variety of purposes, such as to provide hemostasis at or near the fistula site; remove tissue in or around the fistula site; improve long-term patency of the fistula; and combinations thereof.

Handle 40 is attached, via connection port 45, to cable 201, which is attached at its other end to a source of energy, RF energy source 200. RF energy source 200 is preferably an RF energy generator configured to deliver RF energy in various modes, such as monopolar and bipolar modes, and with various adjustable energy settings such as power, frequency, amplitude, and other energy parameter settings. These settings can be visualized and varied through one or more visual displays or controls of user interface 210. RF energy source 200 is attached to ground pad 205, preferably a conductive dispersive pad attached to the back of the patient, when the catheter is configured to deliver monopolar RF energy with electrodes 22.

In a preferred embodiment, monopolar energy or bipolar energy via the two electrodes 22 depicted on opposite sides of conical tip 21. Bipolar energy delivery results in more precise, shallow ablations while monopolar delivery results in deeper ablations. Initiation of energy delivery is accomplished by an operator through depression of button 47, an electrical switch that provides signal communication with RF energy source 200. Energy can be delivered in a closed loop fashion, such as a system with temperature feedback wherein the detected temperature causes an automatic modification of the type, frequency and or magnitude of the energy delivered. In an alternative embodiment, other forms of energy can be used by the catheter apparatus individually or in combination, such forms of energy selected from the group consisting of: sound energy such as acoustic energy and ultrasound energy; electromagnetic energy such as electrical, magnetic, microwave and radiofrequency energies; thermal energy such as heat and cryogenic energies; chemical energy; light energy such as infrared and visible light energies; mechanical energy; radiation; and combinations thereof.

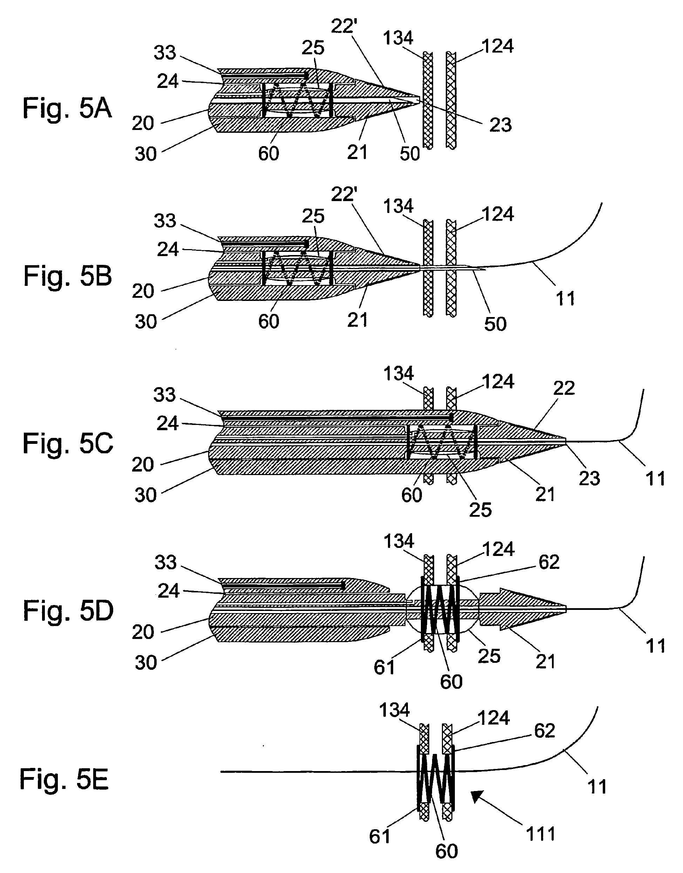

Handle 40 includes a rotating knob, knob 46 which can be used to change one or more parameters of RF energy source 200 or can be operably attached to a pull-wire attached to a distal portion of inner core 20, pull wire 33 (Figs. 5a-5d), to controllably deflect the distal portion of outer sheath 30 and/or inner core 20. Handle 40 further includes inflation port 41, which is fluidly connected to a balloon 25, integral to a distal portion of inner core 20 as depicted in Fig. 1a.

Referring now to Fig. 1a, a sectional view of the distal portion of catheter apparatus 100 of Fig. 1 is illustrated with guidewire 11 removed. Inner core 20 is shown inserted within the lumen of outer sheath 30, such that flange 29 or the conical tip 21 is in contact with the distal end of outer sheath 30 preventing inner core 20 from being further retracted. Two electrodes 22 are shown on opposite sides of conical tip 22 such that bipolar energy can be delivered from one electrode to the other. Alternatively, monopolar energy can be delivered to each electrode 22 either independently or simultaneously. The geometry and construction of the electrodes on the distal tip 21 may vary significantly. In addition to opposed electrode pairs (as illustrated) the electrode structure may comprise a single ring electrode formed around the conical tip, a plurality of axial electrodes formed on the surface of the conical tip, a mesh or other surface electrode structure formed over the conical tip, or the like. Conical tip 21 includes guidewire lumen 23, such that a guidewire can be inserted and exit a port located on a handle attached to the proximal end of inner core 20.

Located within a recess located near the distal end of inner core 20 is an expandable structure, anastomotic clip 60, which is configured to be placed between the two anatomical structures and assist in maintaining the fistula in a fluidly open state. Clip 60 can be constructed of one or more biocompatible materials or coatings including but not limited to: shape memory alloys, such as nickel-titanium alloys (Nitinol™; Elgiloy™); stainless steel; gold; platinum; platinum iridium; polymers; elastomers; memory shaped polymers; polytetrafluoroethylene; hydrophilic coatings; hydrophobic coatings; pharmaceutical agent coatings; and radiopaque coatings. Clip 60 may be self-expanding, balloon expandable, or include both self-expanding and balloon expandable portions. Clip 60 can provide functions selected from the group consisting of; providing hemostasis in or around the fistula tissue; reducing neointimal proliferation into the fistula; exerting radial force on the tissue surrounding the fistula; acting as a radioactive source to deliver radiation to the tissue surrounding the fistula; acting as a drug delivery depot delivering one or more agents to the tissue of the fistula; and combinations thereof. In a preferred embodiment, clip 60 is also configured as an electrode that is attached to one or more electrical wires to deliver energy, such as monopolar or bipolar energy, to the tissue surrounding the fistula. In general, clip 60 can provide functions to enhance the resultant therapeutic benefit of the fistula and/or prevent or reduce undesired side effects such as thrombus or atheroma formation, neointimal proliferation, vessel erosion and other adverse conditions.

Located beneath clip 60 at the recessed portion of inner core 20 is an expandable balloon 25 which is configured similar to balloons or stent delivery catheters. Balloon 25 may be a compliant or a non-compliant balloon. Balloon 25 may have a singular diameter when expanded, or may have a varying radial profile such as to expand different portions of the fistula and/or clip 60 to different diameters or shapes. In a preferred embodiment, balloon 25 is configured to apply force in a radial direction within the fistula, as well as other directions of force such as to deform portions of clip 60 against the walls of either or both anatomical structures. Balloon 25 is fluidly connected via an inflation lumen within inner core 20, inflation lumen 24, to inflation port 41 of handle 40, both depicted in Fig. 1, such that an endoflator or other inflation device may be used to precisely inflate balloon 25 at a controlled inflation rate to a specific pressure.

The anastomotic clip 60 can be delivered in a variety of ways. For self-expanding clips, for example formed from shape or heat memory alloys, deployment may be achieved by simply retracting the outer sheath 30 to release the clip into the fistula. The placement of the self-expanding clip 60 can be modified or embedded by inflation of the balloon 25. Alternatively, the balloon 25 may be used directly for expansion of clips 60 formed from malleable materials, such as cobalt chrome alloys. In both cases, placement of the clip 60 within the fistula may be further modified by delivering ablation or other energy through or beneath the clip. For example, the clip could be connected to a regular frequency or other power source to deliver the desired ablation or other energy. Alternatively, the balloon 25 could include electrode structures (not shown) to deliver radiofrequency or other energy through the deployed clip 60.

Referring now to Figs. 1B through 1D, the geometry of the tip 21 may be varied. For example, a tip structure 21' may be modified to have a tapered geometry where the diameter decreases in the distal direction, as shown in Fig. 1B. Fig. 1B also shows alternative ablation element configurations where four orthogonally opposed electrodes 22a are orthogonally arranged on the tapered tip 21a. Additionally, electrodes 22b may also be arranged near the distal end of the outer sheath 30. In Fig. 1C, the conical tip 21b is arranged to have a bullet profile with a pair of opposed electrodes 22c on its outer surface. In Fig. 1D, tapered tip 21c has an asymmetric profile with its tip aligned with the outer periphery of the outer sheath 30. A first mesh electrode structure 22d is located on the tapered tip 21c and a second mesh electrode structure 22e is located at the distal end of the outer sheath 30. It will be appreciated that the different distal tip geometries and electrode configurations can be employed in a wide variety of specific combinations.

Referring to Fig. 2 an initial step in creating an aorto-caval fistula is illustrated. Depicted in Fig. 2 is aorta 130, which bifurcates into right arterial iliac 132 and left arterial iliac 133. Also depicted is IVC 120 which bifurcates into right venous iliac 122 and left venous iliac 123. An introducer sheath, venous introducer 125, is placed in the groin area of the patient, into a right femoral vein to provide access to right venous iliac 122. A venous catheter 70 may be used for injecting contrast medium and other agents, as well as the passage of guidewires and other devices through an inner lumen, after the placement through venous introducer 125 so that the catheter tip resides inferior to the intended location for the fistula, fistula site 111. This tip location allows radiographic dye or other contrast medium injected through catheter 41 to travel, with the venous blood flow, past fistula site 111 and toward the heart of the patient.

A second introducer sheath, arterial introducer 135, is placed in the groin area of the patient into the left femoral artery. Catheter apparatus 10 is inserted through arterial introducer 135 and advanced to a location proximate fistula creation site 111. In a preferred embodiment, apparatus 10 is multi-functional and provides functions selected from the group consisting of: injection of contrast medium including radiographic dyes and ultrasonic medium; injection of drugs or other agents such as through an integral needle; aspiration of blood; vessel to vessel needle advancement and vessel to vessel guidewire introduction through the needle; visualization of internal structures such as via an integral or inserted ultrasound catheter; fistula and/or implant dilation such as via an integral balloon; tissue debulking such as via integral ablation electrodes; placement of an anastomotic clip; removal of an anastomotic clip; placement of a fistula treatment device; placement of a anastomotic clip treatment device; placement of a flow modification device; and combinations thereof. These various functions can be performed by or with the assistance of apparatus 10 through the use of functional elements integrated into apparatus 10, or separate devices (third elongate elements) which can be passed through one or more lumens accessible from the proximal end of apparatus 10.

Located at the proximal end of apparatus 10 are various controls (not shown) that are used to rotate, advance, retract, manipulate, activate, or otherwise control the slidable tubes, needles and other elements of apparatus 10. Sheath advancement hub 42 is connected to outer sheath 30, which surrounds various internal tubes, elements and lumens. In a preferred embodiment, apparatus 10 includes a visualization element, such as an ultrasound element, not shown. The visualization element can produce, through electronic or other means, a visual representation of the device and neighboring tissue. The visualization element may be an ultrasound catheter, such as a rotational or fixed array ultrasound catheter, which creates a cross-sectional image of the area surrounding the device. The ultrasound catheter can be inserted into one or more lumens of apparatus 10. Alternatively, catheter apparatus 10 includes an integrated ultrasound element, not shown. The ultrasound element comprises an array of ultrasound crystals that are circumferentially mounted along a distal portion of apparatus 10 and are electrically connected to an attachment port on the proximal end of apparatus 10, the attachment port connecting to an ultrasound monitor, all not shown. In an alternative or additional, preferred embodiment, apparatus 10 may include one or more visualization markers, such as radiographic markers or embedded agents such as barium sulfate, or ultrasonically visible markers, all not shown. These markers can be used to perform controlled advancements, retractions, rotations and other positioning of apparatus 10 during the fistula creation procedures utilizing the appropriate visualization means.

The second elongate tube, inner core 20, is slidingly received within outer sheath 30, and has at its distal end conical tip 21, which preferably has a dilating tip shape, and includes one or more electrodes, described in detail in reference to Fig. 1. Tip 21 is advanced to the intended fistula site 111, by advancing apparatus 10 through arterial introducer 135 while advancing core advancement hub 43, located on the proximal end of inner core 20. Within a lumen of core 20 is another elongate tube 50, such as a flexible, advanceable needle, which has attached at its proximal end, needle advancement hub 44. Slidingly received within needle 30 is a standard interventional guidewire, guidewire 11. Needle 50 may consist of an outer protective sheath, not shown, with a flexible, advanceable needle contained within its lumen.

Tip 21 is positioned against the wall of aorta 130 such that the distal end of needle 50 can be advanced from aorta 130 into IVC 120. In an alternative embodiment, tip deflecting means are integral to outer sheath 30 and/or inner core 20, to direct needle 50 at a near 90 degree angle to the wall of aorta 130. In another alternative embodiment, catheter apparatus 10 is inserted into a deflectable guide catheter to cause the needle to be properly oriented. In another alternative, preferred embodiment, the procedure is performed from vein to artery, such as from IVC 120 to aorta 130.

Referring back to Fig. 2, prior to advancing needle 50 out of tip 21, radiographic dye can be injected to visualize the border of the starting vessel, aorta 130, under fluoroscopy. An injection of contrast medium from imaging catheter 70, can be performed to visualize the border of the IVC 120 walls as well. Additionally, contrast medium can be injected through the lumen in needle 50, or via a separate lumen incorporated through the length of catheter apparatus 10. In an alternative, preferred method, another introducer sheath is placed in the groin area of the patient and into the right femoral artery and a pigtail catheter is inserted through the arterial introducer and advanced to a location superior to fistula creation site 111, the right femoral artery introducer sheath and pigtail catheter not shown. Contrast medium injections can be performed through the pigtail catheter, such that arterial flow can be visualized under fluoroscopy prior to, during and after fistula creation, for anatomical landmarking including but not limited to: location of vessel walls, sizing of vessel and fistula lumens, estimation of blood flow and other purposes.

In the artery to vein approach depicted in Fig. 2, the outer sheath 30 can be positioned against the medial aortic wall to provide support as needle 50 is advanced. Typical advancement distance of needle 50 is at least 5 mm, which can be controlled with markings or other control means located on the proximal end of catheter apparatus 10, markings and control means not shown. After the needle 50 is advanced, or partially advanced, a contrast medium injection can be performed through the lumen in needle 50, to confirm access of the target vessel, IVC 120. In an alternative embodiment of the catheter apparatus of Fig. 2, needle 50 is inserted into the lumen of outer sheath 30 when inner core 20 is removed.

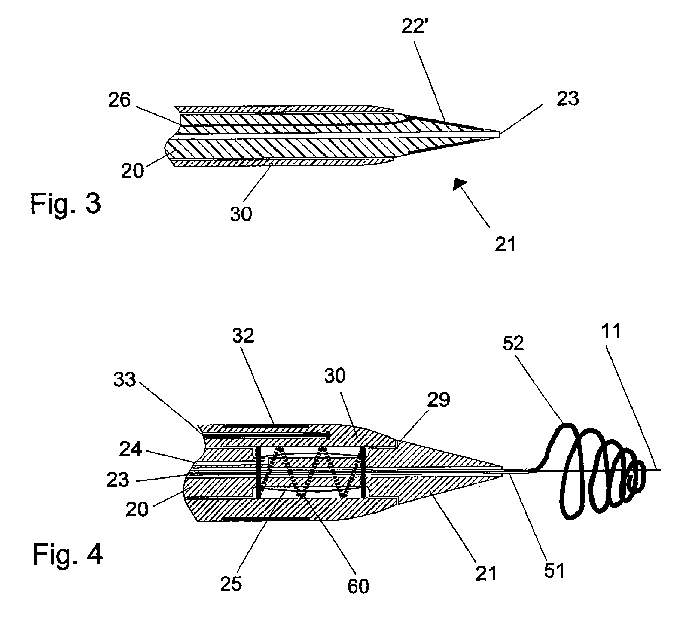

Referring now to Fig. 3, another exemplary embodiment of the catheter apparatus is illustrated, depicting a sectional view of the distal portion of the catheter apparatus. Outer sheath 30, described in detail hereabove, has a tapered distal end that provides a smooth transition with conical tip 21 of inner core 20 when positioned as shown. Inner core 20 and conical tip 21 do not include the flange included in the embodiment of Fig. 1a, such that the inner core 20 can be retracted proximally and completely withdrawn from the lumen of outer sheath 30, allowing the insertion of one or more additional tubular conduits, such as an IVUS catheter, balloon catheter, anastomotic clip delivery catheter, ablation catheter, or other catheter or similar device. In a preferred embodiment, an alternative configuration of the inner core, such as an inner core with a different profile conical tip, or a different type of ablation element such as an ultrasound energy or light energy ablation element, can be subsequently or alternatively inserted into the lumen of sheath 30. In a preferred system embodiment, a kit including multiple inner cores of different configurations are supplied, such as with a single outer sheath.

Referring back to Fig. 3, inner core 20 includes a lumen from the proximal end to the distal end, guidewire lumen 23, such that inner core 20 can be delivered over the wire, such as a wire passing from an artery to a vein. Subsequent devices inserted through the lumen of outer sheath 30 may also be delivered over the same or a different guidewire. Conical tip 21 further includes a cone shaped electrode, electrode 22, which is electrically attached to wiring 26 which travels proximally to a connection port, not shown but located on the proximal end of the catheter apparatus of the present invention, such that a connection can be made to a supply of energy such as RF energy.

Referring now to Fig. 4, another preferred embodiment of the catheter apparatus is illustrated, depicting a sectional view of the distal portion of the catheter apparatus. Outer sheath 30, described in detail hereabove, has a tapered distal end that provides a smooth transition with conical tip 21 of inner core 20 when inner core 20 is fully retracted. Inner core 20 and conical tip 21 include flange 29, which prevents inner core 20 from being further withdrawn into the lumen of outer sheath 30. Inner core 20 also includes lumen 23, which permits the insertion of various elongate tubes, such as guidewire and catheter devices. Shown inserted into lumen 23 is ablation shaft 51, an advanceable, flexible, elongate tube, which terminates in coil tip 52. Coil tip 52 is preferably a resiliently biased structure that assumes a spiral shape when in its expanded state. Coil tip 52 may include one or more electrodes, not shown but preferably platinum bands, mounted to and electrically isolated from the resiliently biased spiral. Alternatively, coil tip 52 functions as a single electrode in its entirety or near entirety. Coil tip 52 is preferably constructed of nickel-titanium alloy and when retracted, can be constrained within lumen 23 of inner core 20. Shaft 51 and spiral tip 52 are sized and configured to be completely removed from lumen 23. In addition, shaft 51 includes a lumen from its proximal end to its distal end such that shaft 51 can be inserted over guidewire 11 and guidewire 11 can be inserted into or removed from the lumen of ablation shaft 51.

Coil tip 52 is electrically attached to a supply of electrical energy, such as via wires that reside within the outer surface of ablation shaft 51 and travel proximally to an electrical connection port. Coil tip 52 may act as a single electrode, such as an electrode which works with an external patch electrode attached on the back of the patient to deliver monopolar RF energy. Alternatively, coil tip 52 may include multiple electrodes to each deliver monopolar RF energy and/or in combination deliver bipolar energy. Shaft 51 can be advanced, such as via controls, not shown, on the proximal end of the catheter apparatus when conical tip 51 is in relative proximity to the proposed fistula creation site, such as when guidewire 11 has already been advanced from a first, or starting anatomical structure to a second, or target anatomical structure. Delivery of energy to tissue can be used to create the fistula and/or to assist in allowing conical tip 21 to pass from the starting structure, such as a vein, to the target structure, such as an artery. Coil tip 52 can be advanced, and energy applied, when an unsuccessful attempt at passing conical tip between two structures has been performed. In a preferred method, coil tip 52 is advanced after a fistula, or a partial fistula has been created, such as to improve flow, to increase fistula diameter, and/or to remove tissue that may be protruding into the flow path of the fistula.

Referring back to Fig. 4, outer sheath 30 may include an ablation element, circumferential electrode 32, which is a continuous conductive band, such as a platinum band, around a small longitudinal segment of the outer wall of outer sheath 30. Circumferential electrode 32 can deliver energy in monopolar mode, or in bipolar mode such as in the transmission of energy to or from spiral tip 52. Similarly, circumferential electrode 32 is electrically attached to one or more wires which travel proximally, within the outer surface of outer sheath 30, and have connection means, not shown, which electrically connect to a source of electrical energy such as an RF energy delivery unit described in detail in reference to Fig. 1. Circumferential electrode 32 can be used for various functions including but not limited to: increase fistula flow, increase fistula diameter, cauterize the fistula area, such as to stop bleeding, and/or remove tissue that may be protruding into the flow path of the fistula. Both spiral tip 52 and circumferential electrode 32 may apply energy before and/or after a scaffolding device or other implant, such as the anastomotic clip are in place. In an alternative embodiment, circumferential electrode 32 extends over a partial portion of the circumference. In another alternative embodiment, circumferential electrode 32 consists of multiple electrodes, spanning the majority of the circumference or less than a majority, such that energy can be delivered between the multiple electrodes in a bipolar mode.

Referring back to Fig. 4, outer sheath 30 further includes pull wire 33, which is fixedly attached to a distal portion of outer sheath 30. Pull wire 33 extends proximally, such as to a handle, not shown, the handle including one or more controls that allow tensioning of pull wire 33 to cause the distal portion of outer sheath 30 to controllably curve such as to point the distal portion of the catheter toward the wall of the starting anatomical structure, such as the wall of the aorta or the wall of the IVC. Located near the distal end of inner core 20 but proximal to conical tip 21 is a recess in which is located anastomotic clip 60, which can be advanced between the openings between the two anatomical structures and delivered into the fistula. Clip 60 is configured to prevent leakage of blood outside of the fistula and the vascular system, and/or to improve long-term patency of the fistula. Anastomotic clip 60 may be self-expanding, may be balloon expandable, or may include both self-expanding and balloon expandable portions. Located on the recess of inner core 20 and under clip 60 is an expandable balloon, balloon 25, which may be a compliant or non-compliant balloon. Balloon 25 is fluidly attached to inflation lumen 24, which extends proximally, to an inflation port, not shown, but described in detail in reference to Figs. 1 and 1a. Balloon 25 may be inflated in the initial delivery of clip 60 into the fistula, and/or after clip 60 is in place, such as to allow further expansion of clip 60. Balloon 25 may be inflated after energy has been delivered from spiral tip 52, circumferential electrode 32, or both.

Referring now to Fig. 5a through Fig. 5e, the apparatus in accordance with the invention is illustrated. Fig. 5a shows an initial step in which a catheter apparatus of the present invention has been transluminally advanced to a location and oriented such that its distal end is toward the wall of a first, or starting anatomical structure at a location wherein the therapeutic fistula is to be created. The catheter apparatus includes outer sheath 30, which includes pull wire 33, described in detail hereabove, and tensioned to cause the desired deflection. Slidingly received within outer sheath 30 is inner core 20, which includes on its distal portion conical tip 21, positioned in close proximity to arterial wall 134. In a preferred embodiment, conical tip 21 includes a bevel cut on its distal end to assist in passing through tissue such as arterial wall 134 and venous wall 124. Conical tip 21 includes cone shaped electrode 22', which provides ablation, cut and/or coagulation energy to tissue, to assist in allowing devices to pass through arterial wall 134 and venous wall 124, as well as modify the fistula to be created.

Extending from the proximal end, not shown, of inner core 20 to the distal end of conical tip 21 is lumen 23, sized to allow guidewires, or other elongate tubes, such as flexible needle assemblies, to be passed therethrough. Residing within lumen 23 is needle 50, which includes advancement and retraction means, not shown, on the proximal end. In a preferred embodiment, advancement and retraction means include visual indicators of distance traveled by needle 50. Located near the distal end of inner core 20 but proximal to conical tip 21 is a recess in which is located anastomotic clip 60. Clip 60 can be advanced to reside between the openings of the two anatomical structures and subsequently delivered into the fistula . Clip 60 is configured to prevent leakage of blood outside of the fistula and the vascular system, and/or to improve long-term patency of the fistula. Anastomotic clip 60 may be self-expanding, may be balloon expandable, or may include both self-expanding and balloon expandable portions. Located on the recess of inner core 20 and under clip 60 is an expandable balloon, balloon 25, which may be a compliant or non-compliant balloon. Balloon 25 is fluidly attached to inflation lumen 24, which extends proximally, to an inflation port, not shown, but described in detail in reference to Figs. 1 and 1a. Balloon 25 may be inflated in the initial delivery of clip 60 into the fistula, and/or after clip 60 is in place, such as to allow further expansion of clip 60 and/or the tissue surrounding clip 60.

Referring now to Fig. 5b, needle 50 has been advanced to first penetrate arterial wall 134 and then penetrate venous wall 124 such that it resides within a lumen of the target vein. Guidewire 11 has been advanced out of needle 50 such that guidewire 11 is advanced further into the lumen of the target vein, either upstream or downstream of normal venous flow. The distal end of conical tip 21 remains in relative contact with arterial wall 134. Referring now to Fig. 5c, needle 50 has been retracted within lumen 23 of inner core 20 while maintaining or potentially advancing guidewire 11. Contrast media may optionally be introduced from the lumen 23 (or other lumen or passage) to allow confirmation that the tip 21 had reached the target vein or other secondary anatomical structure. Release of contrast into venous circulation is easy to detect. Inner core 20 and outer sheath 30 are advanced, over-the-wire, such that inner core 20 and outer sheath 30 pass through arterial wall 134 and venous wall 124. During the advancement step, in a continuous, stopped, or intermittent fashion, energy may be applied to conical electrode 22 to assist in the advancement process, prevent undesired bleeding, and/or provide another function described in detail hereabove. Inner core 20 is positioned such that clip 60 and balloon 25 are relatively centered between the two vessel walls, arterial wall 134 and venous wall 124.

Referring now to Fig. 5d, outer sheath 30 has been retracted, maintaining inner core 20 and guidewire 11 in the same position. Clip 60 has self-expanded, and balloon 25 has been inflated to further expand clip 60. In an alternative embodiment, balloon 25 expands clip 60 for the initial expansion. In either embodiment, clip 60 may include both self-expanding and balloon expandable portions. Proximal flange 61 is in contact with arterial wall 134 and distal flange 62 is in contact with venous wall 124 such that clip 60 can provide a tensional force between the two vessels. The mid-portion of clip 60 is configured to provide radial force to the tissue of the two vessel walls, and any tissue in between. The mid-portion of clip 60 includes sufficient scaffolding to reduce protrusion of tissue into the tube shaped flow path provided. In an alternative embodiment, one or more electrodes are included on the outer surface of balloon 25, electrodes and attaching wires not shown, such that monopolar and/or bipolar energy can be applied to the tissue surrounding clip 60. In another alternative embodiment, clip 60 is attached to detachable wires, wires not shown but extending proximally to an attached energy source, such that monopolar or bipolar energy can be applied to the tissue surrounding clip 60 by clip 60 itself. In another alternative embodiment, clip 60 includes multiple electrodes, such as multiple platinum bands connected to a supply of energy, the platinum bands providing energy to the surrounding tissue when clip 60 is in place.

Referring now to Fig. 5e, inner core 20 and outer sheath 30 have been retracted and removed from the body of the patient, leaving guidewire 11 in place. Fistula 111 includes the scaffolding and tensioning structure, clip 60, which supports long-term flow of oxygenated blood from the artery to the vein. Proximal flange 61 and distal flange 62 of clip 60 make contact with arterial wall 134 and venous wall 124 respectively, such as to provide a tensioning, as well as an aligning force between the two vessels. Leaving guidewire 11 in place, or re-inserting guidewire 11 through the fistula, allows additional devices to be passed through the vascular, through clip 60 and the fistula, and into the vein receiving the oxygenated blood from the artery. These additional devices can be selected from the group consisting of: a balloon catheter such as a balloon catheter used to increase the diameter of the clip and/or the fistula; a drug delivery catheter such as a balloon eluding drug delivery catheter used to deliver one or more agents to the clip and/or the fistula; a radiation delivery catheter such as a radiation delivery catheter used to reduce neointimal proliferation into the fistula; an energy delivery catheter such as an energy delivery catheter used to apply energy to tissue protruding into the fistula; a cutting device such as a pull-back cutter used to remove tissue that is protruding into the fistula; a manipulating arm such as a manipulating arm used to reposition and/or reconfigure the anastomotic clip; a clip delivery device such as a catheter used to place a second anastomotic clip; and combinations thereof

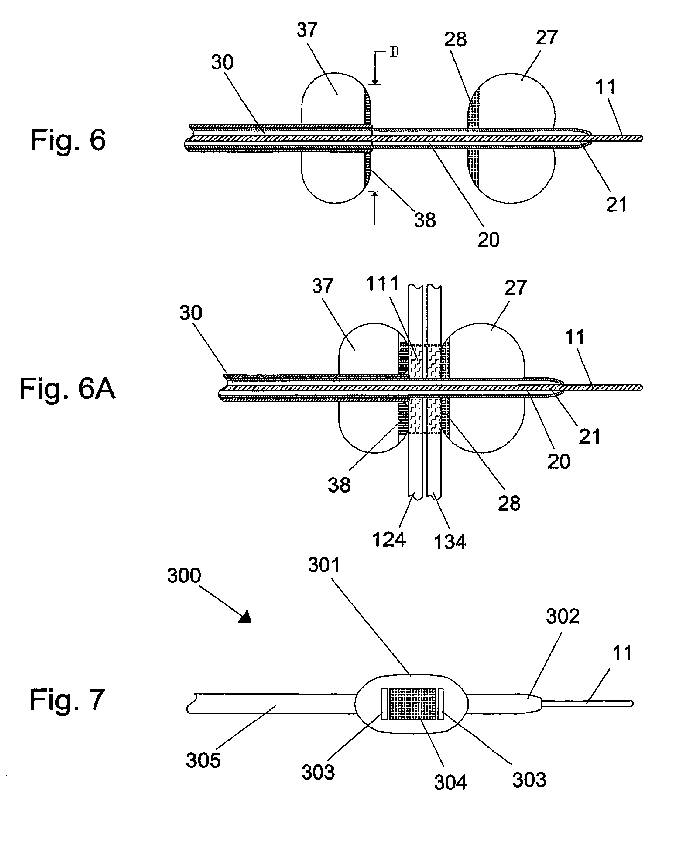

Referring now to Fig. 6, a sectional distal view of another aspect of the catheter apparatus is illustrated. The catheter apparatus includes a first elongate tube, outer sheath 30, which is constructed of materials that are biocompatible and support advancement and torque requirements of transluminal catheter procedures. Located on the outer surface of the distal end of outer sheath 30 is an expandable balloon, balloon 37, which may be a compliant or a non-compliant balloon. Located on the distal surface of balloon 37 is electrode 38. Electrode 38 may be constructed of a metal foil, such as aluminum or gold foil, or may be a conductive coating, such as a metal deposition coating. Wires, not shown but extending to the proximal end of the catheter apparatus of Fig. 6, attach to an energy delivery apparatus such as an RF generator.

Slidingly received within a lumen of outer sheath 30 is inner core 20, which is also constructed of materials that are biocompatible and support advancement and torque requirements of transluminal catheter procedures. Located near the distal end of inner core 20 is distal balloon 27, also an expandable compliant or non-compliant balloon. Located on the proximal surface of distal balloon 27 is electrode 28, which also may be constructed of a metal foil or a conductive coating. Wires, also not shown but extending to the proximal end of the catheter apparatus, attach to the same RF generator. Inner core 20 includes a tapered, dilating end, conical end 21, and a thru lumen such that the catheter apparatus can be inserted over guidewire 11. The diameter of electrode 38 and electrode 28 are similar and chosen to create a fistula of a relative diameter, D. Referring now to Fig. 6a, a sectional view of the catheter apparatus of Fig. 6 is shown wherein inner core 20 has been placed over guidewire 11 such that conical tip 21 and balloon 27, in a deflated state as controlled from the proximal end, penetrate through venous wall 124 and then arterial wall 134. In an alternative, preferred embodiment, the penetration is made from arterial wall 134 and then venous wall 124. In another preferred embodiment, conical tip 21 includes penetration assisting means such as a bevel cut tip and/or a conical electrode configured to ablate tissue. As shown in Fig. 6a, balloon 27 has been inflated and inner core 20 has been retracted such that electrode 28 of balloon 27 is contact with arterial wall 134. Balloon 37 is inflated and outer sheath 30 has been advanced such that electrode 38 of balloon 37 is in contact with venous wall 124. Bipolar energy is then applied between electrode 28 of distal balloon 27 and electrode 38 of proximal balloon 37 such that the tissue between the balloons is ablated, creating fistula 111, such as a fistula created to divert blood from the arterial system to the venous system, to reduce systemic vascular resistance, provide oxygenated blood to increase to oxygen content of blood supplied to the lungs, increase cardiac output, and provide other therapeutic benefits.

Referring now to Fig. 7, a sectional side view of another aspect of the catheter apparatus is illustrated. Ablation catheter 300 includes shaft 305, which is constructed of materials that are biocompatible and support advancement and torque requirements of transluminal catheter procedures. Located on the distal end of shaft 305 is a tapered, dilating end, conical tip 302, which may include a beveled cutting end and/or or a conical electrode, both not shown but described in detail hereabove. Shaft 305 includes a lumen extending from its proximal end to its distal end, such that ablation catheter 300 can be introduced transluminally over a guidewire, such as a guidewire that has been placed from an artery or a vein through a fistula. Located on the distal end of shaft 305 is an expandable balloon, balloon 301, which may be a compliant or a non-compliant balloon. Located on the distal surface of balloon 301 is an electrode, electrode 304, which covers a partial circumferential surface of balloon 301. Electrode 304 is a preferably a flexible conductive surface, such as a metal foil or a metal coating. In an alternative embodiment, multiple electrodes may be included along the outer surface of balloon 301, such as to create a full circumference, or a specific geometric shape. Adjacent to each end of electrode 304 are radiopaque markers 303, which are used to position balloon 301 and electrode 304 in a fistula, with both longitudinal and rotational orientation. In an examplary method, ablation catheter 300 is used to provide energy to the tissue surrounding a fistula that has been created to provide therapeutic benefit to a patient by decreasing the systemic resistance and/or increasing the oxygen content of the blood supplied to the lungs, and/or increase cardiac output, as has been described in detail hereabove. In another preferred embodiment, ablation catheter 300 is used to provide energy to tissue surrounding an anastomotic clip that has been placed to prevent bleeding and/or provide long-term patency of an arteriovenous fistula created for the similar therapeutic benefits.

It should be understood that numerous other configurations of the systems, devices and methods described herein may be employed without departing from the scope of this application. The catheter apparatus may include tip-deflecting means, as has been described hereabove, or may be used in conjunction with a steerable sheath. Each of the elongate tubes may include one or more lumens, such as lumens to accept other catheters and catheter devices, as well as guidewires and needle assemblies. Each of the lumens may include a lining, such as a Teflon lining placed on the inner surface of a needle lumen through which a guidewire is inserted. Additional lumens, such as blind lumens that have an entry point but terminate in a dead end not exiting the end of the tube, can be included in one or more elongate tubes such as to accept an IVUS catheter used to produce a cross-sectional image of the fistula and/or neighboring tissue. Alternatively, each elongate tube may include a rapid exchange sidecar at or near its distal end, consisting of a small projection extending radially outward from an outer wall of the elongate tube, with a guidewire lumen through the projection along the axis of the tube. Each shaft may include a balloon, such as a compliant or non-compliant balloon. These balloons may be concentric with the shaft, or eccentric with the shaft. Each balloon may include one or more ablation elements, such as flexible electrodes, to deliver energy to tissue surrounding the fistula, either from within the fistula or at a close proximity solely within a single vessel. Each elongate tube may include a handle integral to their proximal end. The handle may include controls for advancement and retraction of inner tubes. The handle may include means of activating the supply of energy, and may include one or more visual indicators that provide information to the operator of the apparatus, such as light emitting diodes or other visual indicators that may work with a battery integral to the handle as well.

The ablation elements can take many forms, including the electrical energy delivery conductive electrodes described in detail throughout this application. Other forms of energy will require different forms of transducers and energy transfer means, and conduits to provide the energy to the ablation element. For example, a cryogenic fluid can be delivered to a thermally conductive surface via a fluid tube. A radiation pellet can be advanced through a lumen to reside within a fenestrated shielding chamber. Sound energy can be delivered to transmitting devices attached to sound propagating conduits. Other forms of ablation elements and energy transmission conduits can be employed without departing from the scope of the application. In a preferred embodiment, the ablation elements are configured to deliver more than one type of energy such as RF energy and ultrasound energy. The energy delivered by the apparatus can be delivered before, during and/or after the dilation of the fistula. The energy can also be delivered before, during, and/or after the placement of an anastomotic clip. The energy can be delivered after an assessment has been made, such as an assessment of flow or an assessment of therapeutic benefit, wherein the current conditions are assessed to be sub-optimal.

Other embodiments of the invention will be apparent to those skilled in the art from consideration of the specification and practice of the invention disclosed herein. It is intended that the specification and examples be considered as exemplary only, with a true scope of the invention being indicated by the following claims. In addition, where this application has listed the steps of a method or procedure in a specific order, it may be possible, or even expedient in certain circumstances, to change the order in which some steps are performed, and it is intended that the particular steps of the method or procedure not be construed as being order-specific unless such order specificity is expressly stated.

## Claims

1. A catheter apparatus for inserting a guidewire between two anatomical structures, said catheter apparatus comprising:
a needle (50) which is advanceable through the anatomical structures to form a fistula therebetween;
a first elongate tube (30) with a proximal end, a distal end, and a lumen (23) therebetween;
a second elongate (20) tube with a proximal end, a distal end, said second elongate tube (20) sized and configured to be slidingly advanced and retracted within the lumen of the first elongate tube (30) wherein the distal end of the second elongate tube (20) has a conical geometry (21);
an ablation element (22') on the conical distal end of the second tubular member (20) for modifying the fistula between the two anatomical structures, **characterised by** the needle (50) having a lumen therethrough adapted to receive the guidewire (11) and the second elongated tube having a lumen therebetween adapted to receive the needle (50).

2. The apparatus of claim 1, wherein the ablation element comprises one or more electrodes (22').

3. The apparatus of claim 1 or 2, wherein the first elongate tube (30) includes a deflectable portion at or near its distal end.

4. The apparatus of claim 3, wherein the distal portion is deflecteable by a pull wire (33) attached to said distal portion.

5. The apparatus of any one of the preceding claims, wherein the second elongate tube (20) is completely removable from the lumen of the first elongate tube (30).

6. The apparatus of any one of the preceding claims further comprising an anastomotic clip (60) mounted near the distal end of the second elongate tube (20).

7. The apparatus of claim 6 wherein the anastomotic clip (60) is mounted in a recess in the outer surface of the second elongate tube (20).

8. The apparatus of claim 7 wherein the anastomotic clip (60) includes a radially expanding portion.

9. The apparatus of claim 8, further comprising a balloon (25) mounted near the distal end of the second elongate tube (20).

10. The apparatus of claim 9, wherein the balloon (25) is mounted under the anastomotic clip (60).

11. The apparatus of claim 6, 7, 8, 9 or 10, wherein the anastomotic clip (60) is configured to provide at least one of (1) tension between walls of the two anatomical structures; (2) hemostasis in or around the fistula tissue; (3) tension between the two anatomical structures; (4) a reduction in neointimal proliferation into the fistula; (5) a radial force on the tissue surrounding the fistula to maintain a lumen between the structures; (6) a radioactive source to deliver radiation to the tissue surrounding the fistula; (7) a drug delivery depot delivering one or more agents to the tissue of the fistula to enhance long-term patency or biocompatibility; and (8) a control means to adjust the flow of blood through the fistula.

12. The apparatus of claim 6, 7, 8, 9 or 10, wherein the anastomotic clip (60) is configured to provide a control means to adjust the flow of blood through the fistula via independent means or in combination with a separate device.

13. The apparatus of claim 6, 7, 8, 9, 10, 11 or 12, wherein the anastomotic clip (60) comprises an electrode to deliver energy to the tissue surrounding the fistula.

14. The apparatus of any of claims 1 to 4, further comprising an expandable balloon (25) which is inflatable within the fistula after the ablation element (22') enlarges said fistula, and an anastomotic clip (60), said clip placeable in the fistula after the balloon enlarges said fistula.

15. The apparatus of any one of the preceding claims wherein the ablation element (22') is adapted to ablate tissue protruding into the fistula.

16. The apparatus of any one of the preceding claims, further comprising a handle (40) mounted on the proximal end of at least one of the first and second elongate tubes (30 and 20), and further comprising one or more controls used to initiate energy delivery from the ablation element (22') and deflection of the distal end of the first elongate tube (30).

17. A system comprising the apparatus of any one of the preceding claims and an energy source (200).

## Patentansprüche

1. Kathetervorrichtung zum Einführen eines Führungsdrahts zwischen zwei anatomische Strukturen, wobei die Kathetervorrichtung aufweist:
eine Nadel (50), die durch die anatomischen Strukturen vorgeschoben werden kann, um dazwischen eine Fistel auszubilden;
eine erste längliche Röhre (30) mit einem proximalen Ende, einem distalen Ende und einem dazwischenliegenden Lumen (23);
eine zweite längliche Röhre (20) mit einem proximalen Ende und einem distalen Ende, wobei die zweite längliche Röhre (20) so dimensioniert und konfiguriert ist, dass sie in dem Lumen der ersten länglichen Röhre (30) gleitend vorgeschoben und zurückgezogen wird, wobei das distale Ende der zweiten länglichen Röhre (20) eine konische Geometrie (21) hat;
ein Ablationselement (22') an dem konischen distalen Ende des zweiten röhrenförmigen Elements (20) zum Verändern der Fistel zwischen den beiden anatomischen Strukturen,
**dadurch gekennzeichnet, dass**
die Nadel (50) ein durchgehendes Lumen hat, das ausgelegt ist, um den Führungsdraht (11) aufzunehmen, und die zweite längliche Röhre ein durchgehendes Lumen hat, das ausgelegt ist, um die Nadel (50) aufzunehmen.

2. Vorrichtung nach Anspruch 1, wobei das Ablationselement eine oder mehrere Elektroden (22') aufweist.

3. Vorrichtung nach Anspruch 1 oder 2, wobei die erste längliche Röhre (30) an oder nahe ihrem distalen Ende einen ablenkbaren Abschnitt aufweist.

4. Vorrichtung nach Anspruch 3, wobei der distale Abschnitt mittels eines Zugdrahts (33), der an dem distalen Abschnitt befestigt ist, ablenkbar ist.

5. Vorrichtung nach einem der vorhergehenden Ansprüche, wobei die zweite längliche Röhre (20) komplett aus dem Lumen der ersten länglichen Röhre (30) entfernbar ist.

6. Vorrichtung nach einem der vorhergehenden Ansprüche, die des Weiteren eine Anastomoseklemme (60) aufweist, die nahe dem distalen Ende der zweiten länglichen Röhre (20) angebracht ist.

7. Vorrichtung nach Anspruch 6, wobei die Anastomoseklemme (60) in einer Ausnehmung in der Außenfläche der zweiten länglichen Röhre (20) angebracht ist.

8. Vorrichtung nach Anspruch 7, wobei die Anastomoseklemme (60) einen sich radial erweiternden Abschnitt aufweist.

9. Vorrichtung nach Anspruch 8, die des Weiteren einen Ballon (25) aufweist, der nahe dem distalen Ende der zweiten länglichen Röhre (20) angebracht ist.

10. Vorrichtung nach Anspruch 9, wobei der Ballon (25) unter der Anastomoseklemme (60) angebracht ist.

11. Vorrichtung nach Anspruch 6, 7, 8, 9 oder 10, wobei die Anastomoseklemme (60) so konfiguriert ist, dass sie wenigstens eines bereitstellt von (1) Zug zwischen Wänden der beiden anatomischen Strukturen; (2) Hämostase in oder um das Fistelgewebe herum; (3) Zug zwischen den beiden anatomischen Strukturen; (4) eine Verringerung der Neointimaproliferation in die Fistel; (5) eine Radialkraft auf das Gewebe, das die Fistel umgibt, um ein Lumen zwischen den Strukturen aufrecht zu erhalten; (6) eine radioaktive Quelle, um Strahlung an das die Fistel umgebende Gewebe zu liefern; (7) ein Medikamentenverabreichungsdepot, das ein oder mehrere Mittel an das Gewebe der Fistel liefert, um die Langzeit-Durchgängigkeit oder Biokompatibilität zu steigern; und (8) ein Steuermittel zum Einstellen des Blutflusses durch die Fistel.

12. Vorrichtung nach Anspruch 6, 7, 8, 9 oder 10, wobei die Anastomoseklemme (60) so konfiguriert ist, dass sie ein Steuermittel bereitstellt, um den Blutfluss durch die Fistel über unabhängige Mittel oder in Kombination mit einer getrennten Vorrichtung bereitzustellen.

13. Vorrichtung nach Anspruch 6, 7, 8, 9, 10, 11 oder 12, wobei die Anastomoseklemme (60) eine Elektrode aufweist, um an das die Fistel umgebende Gewebe Energie zu liefern.

14. Vorrichtung nach einem der Ansprüche 1 bis 4, die des Weiteren einen expandierbaren Ballon (25) aufweist, der in der Fistel aufblasbar ist, nachdem das Ablationselement (22') die Fistel vergrößert hat, und eine Anastomoseklemme (60), wobei die Klemme in der Fistel platziert werden kann, nachdem der Ballon die Fistel vergrößert hat.

15. Vorrichtung nach einem der vorhergehenden Ansprüche, wobei das Ablationselement (22') dazu ausgelegt ist, Gewebe, das in die Fistel vorsteht, zu abladieren.

16. Vorrichtung nach einem der vorhergehenden Ansprüche, die des Weiteren einen Griff (40) aufweist, der an dem proximalen Ende von wenigstens einem der ersten und der zweiten länglichen Röhre (30 und 20) angebracht ist, und die des Weiteren eine oder mehr Steuerungen aufweist, die verwendet werden, um Energiezufuhr von dem Ablationselement (22') und Ablenkung des distalen Endes der ersten länglichen Röhre (30) zu initiieren.

17. System, das die Vorrichtung nach einem der vorhergehenden Ansprüche und eine Energiequelle (200) aufweist.

## Revendications

1. Appareil à cathéter pour insérer un guide-fil entre deux structures anatomiques, ledit appareil à cathéter comprenant :
une aiguille (50) qui peut être avancée à travers les structures anatomiques, afin de former une fistule entre elles ;
un premier tube allongé (30) avec une extrémité proximale, une extrémité distale et une lumière (23) entre elles ;
un second tube allongé (20) avec une extrémité proximale, une extrémité distale, ledit second tube allongé (20) étant dimensionné et configuré pour être avancé et rétracté par coulissement dans la lumière du premier tube allongé (30), dans lequel l'extrémité distale du second tube allongé (20) a une géométrie conique (21) ;
un élément d'ablation (22') sur l'extrémité distale conique du second élément tubulaire (20), permettant de modifier la fistule entre les deux structures anatomiques, **caractérisé en ce que** l'aiguille (50) a une lumière apte à recevoir le guide-fil (11) et le second tube allongé a une lumière, apte à recevoir l'aiguille (50).

2. Appareil selon la revendication 1, dans lequel l'élément d'ablation comprend une ou plusieurs électrodes (22').

3. Appareil selon la revendication 1 ou 2, dans lequel le premier tube allongé (30) comprend une partie pouvant être déviée au niveau de son extrémité distale ou près de celle-ci.

4. Appareil selon la revendication 3, dans lequel la partie distale peut être déviée par un fil de traction (33) fixé à ladite partie distale.

5. Appareil selon l'une quelconque des revendications précédentes, dans lequel le second tube allongé (20) peut être totalement enlevé de la lumière du premier tube allongé (30).

6. Appareil selon l'une quelconque des revendications précédentes, comprenant en outre une pince anastomotique (60) montée près de l'extrémité distale du second tube allongé (20).

7. Appareil selon la revendication 6, dans lequel la pince anastomotique (60) est montée dans une cavité ménagée dans la surface extérieure du second tube allongé (20).

8. Appareil selon la revendication 7, dans lequel la pince anastomotique (60) comprend une partie s'étendant radialement.

9. Appareil selon la revendication 8, comprenant en outre un ballonnet (25) monté sous l'extrémité distale du second tube allongé (20).

10. Appareil selon la revendication 9, dans lequel le ballonnet (25) est monté sous la pince anastomotique (60).

11. Appareil selon la revendication 6, 7, 8, 9 ou 10, dans lequel la pince anastomotique (60) est configurée, de façon à fournir au moins un élément parmi (1) une tension entre les parois des deux structures anatomiques ; (2) une hémostase dans ou autour du tissu de la fistule ; (3) une tension entre les deux structures anatomiques ; (4) une réduction de la prolifération néo-intime dans la fistule ; (5) une force radiale sur le tissu environnant la fistule pour maintenir une lumière entre les structures ; (6) une source radioactive pour fournir des rayonnements au tissu environnant la fistule ; (7) une formulation retard de médicament administrant un ou plusieurs agents au tissu de la fistule afin d'augmenter la perméabilité à long terme ou la biocompatibilité ; et (8) des moyens de contrôle pour ajuster le flux de sang à travers la fistule.

12. Appareil selon la revendication 6, 7, 8, 9 ou 10, dans lequel la pince anastomotique (60) est configurée, de façon à fournir un moyen de contrôle pour ajuster le flux de sang à travers la fistule, par le biais de moyens indépendants ou en combinaison avec un dispositif séparé.

13. Appareil selon la revendication 6, 7, 8, 9, 10, 11 ou 12, dans lequel la pince anastomotique (60) comprend une électrode pour fournir de l'énergie au tissu environnant la fistule.

14. Appareil selon l'une quelconque des revendications 1 à 4, comprenant en outre un ballonnet gonflable (25), qui peut être gonflé dans la fistule après que l'élément d'ablation (22') a élargi ladite fistule, et une pince anastomotique (60), ladite pince pouvant être placée dans la fistule après que le ballonnet a élargi ladite fistule.

15. Appareil selon l'une quelconque des revendications précédentes, dans lequel l'élément d'ablation (22') est adapté pour couper le tissu saillant dans la fistule.

16. Appareil selon l'une quelconque des revendications précédentes, comprenant en outre une poignée (40) montée sur l'extrémité proximale d'au moins un parmi le premier et le second tubes allongés (30 et 20) et comprenant en outre une ou plusieurs commandes, utilisées pour enclencher la distribution d'énergie depuis l'élément d'ablation (22') et la déflection de l'extrémité distale du premier tube allongé (30).

17. Système comprenant l'appareil selon l'une quelconque des revendications précédentes ainsi qu'une source d'énergie (200).
